# EUROPEAN PATENT SPECIFICATION

(11) **EP 2 821 467 B1**
(45) Date of publication and mention of the grant of the patent: **14.02.2018**
(21) Application number: 12869212.6
(22) Date of filing: 24.02.2012
(51) Int. Cl.: C10M 173/02, C10N 10/02, C10N 10/04, C10N 10/06, C10N 10/16, C10N 30/06, C10N 40/00, B29C 33/60

(54) **USE OF A LUBRICITY REGULATING AGENT FOR SILICONE SURFACES**
VERWENDUNG EINES SCHMIERFÄHIGKEITS-REGULIERUNGSMITTELS FÜR SILICONOBERFLÄCHEN
UTILISATION D' UN AGENT RÉGULATEUR LUBRIFIANT POUR LES SURFACES DE SILICONE

(43) Date of publication of application: 07.01.2015
(73) Proprietor: Fain-Biomedical Inc., Aichi 464-0819 (JP)
(72) Inventor: IKEDA, Seiichi, Nagoya-shi Aichi 464-0858 (JP)
(74) Representative: Garavelli, Paolo
(86) International application number: PCT/JP2012/054552
(87) International publication number: WO 2013/125026

(56) References cited:
- EP-A1- 0 795 599
- WO-A1-03/096308
- DE-A1-102009 026 775
- JP-A- H06 261 935
- JP-A- H09 176 677
- JP-A- H11 244 375
- JP-A- 2001 517 712
- JP-A- 2008 158 468
- JP-U- 3 015 310
- US-A- 5 584 943
- DATABASE WPI Week 199049 Thomson Scientific, London, GB; AN 1990-364483 XP002745416, & JP H02 261609 A (SHOWA ELECTRIC WIRE CO LTD) 24 October 1990 (1990-10-24)

## Description

### [Technical Field]

The present invention relates to the use of a lubricity regulating agent for regulating the lubricity of a surface of silicone rubber, and is suitable for use, for example, in a circulating liquid for a catheter simulator using a blood vessel model made of silicone rubber.

### [Prior Art]

Silicone rubber is utilized in various industrial fields as an elastic material, and the lubricity of a surface of silicone rubber must be controlled in some cases.

The present inventors developed and commercialized a catheter simulator which resembles a human shape (see Patent Document 1). In this catheter simulator, a partition member is built in a mannequin main body made of a transparent material so that a blood vessel model as a three-dimensional model is supported by the one surface of the partition member, and an auxiliary instrument for getting the blood vessel model to work is arranged. The blood vessel model is formed of silicone rubber, and the auxiliary instrument is provided with a tank, a pump and a connection pipe. A circulating liquid is contained in the tank, and circulates through the blood vessel model via the connection pipe with a pump. When the catheter is inserted into this blood vessel model, the lubricity of a surface of silicone rubber is an issue to be solved.

See Patent Document 2 as a document which discloses a technique relevant to the present invention.

### [Prior Art Document]

### [Patent Document]

[Patent Document 1] JP 2006-267565 A
[Patent Document 2] JP 09-53064 A

### [Summary of the Invention]

### [Problem to Be Solved by the Invention]

The catheter simulator disclosed in Patent Document 1 enables a catheter to be inserted into a blood vessel model while distributing a circulating liquid through the blood vessel model made of silicone rubber.

While liquids mainly comprising silicone oil (oil-based circulating liquids) and those mainly comprising water (water-based circulating liquids) are used as the circulating liquid, the water-based circulating liquids are preferably used in connection with cost and from the viewpoint of similarity to blood. When mere water is circulated in a blood vessel model, the catheter cannot be smoothly inserted because of its large contact resistance to the internal wall of the blood vessel model.

Therefore, a surfactant as a lubricity regulating agent is mixed with water, thereby making it possible to reduce the contact resistance between the catheter and the internal wall of the blood vessel and to smoothly insert the catheter into the blood vessel model.

However, a blood vessel model in which a circulating liquid obtained by mixing a surfactant with water is circulated differs from an actual blood vessel through which blood circulates. Hence, a simulator using such a circulating liquid inevitably brought an uncomfortable feeling of catheter insertion as compared with the feeling at the time of an actual operation. For example, the contact resistance at a meandering site of the blood vessel model was quite larger than that of an actual operation. Also, when the inserted catheter was allowed to stand for several seconds, such a phenomenon was observed wherein the coefficient of static friction between the catheter and the blood vessel model abnormally increased, leading to the difficulty in pulling out the inserted catheter (which phenomenon is hereinafter referred to as "adhesion").

The use of silicone oil as a circulating liquid does not cause the above problem. However, silicone oil is expensive, and so-called oil-based silicone oil is significantly different in physical properties from blood, and thus reality is lost in the feeling of handling at the time of inserting a catheter.

The present inventors attempted to increase the amount of the surfactant to be incorporated in order to reduce the contact resistance to the internal wall of the blood vessel model, but could not solve the problems of increase in resistance at the meandering site and adhesion. Further, the increase in amount of the surfactant to be incorporated causes slimy feeling in a circulating liquid, and thus is not preferred.

### [Means for Solving the Problem]

The invention relates to a use as claimed in Claim 1.

The present inventor repeatedly made earnest reviews to make the feeling of catheter insertion when using a so-called water-based circulating liquid equal to the feeling of insertion at the time of an actual operation, and, as a result, have found that, upon using a surfactant and a water-soluble ion compound in combination as a lubricity regulating agent, the feeling of insertion of a catheter into a blood vessel model becomes very close to the feeling of catheter insertion at the time of an actual operation. Thus, the insertion resistance does not increase even at a meandering site of a blood vessel model, and little adhesion between the blood vessel model and the catheter would be caused.

A first example is defined as follows: a lubricity regulating liquid for regulating the lubricity of a surface of silicone rubber, comprising water, a surfactant, and a water-soluble ion compound.

The lubricity regulating liquid mainly comprises water, and is obtained by adding, to water, a lubricity regulating agent comprising a surfactant and a water-soluble ion compound and mixing them together. In the meantime, in the case where the surfactant itself is an ion compound, the phrase "water-soluble ion compound" used herein is regarded as referring to a water-soluble ion compound except the surfactant.

The role of the surfactant mainly resides in reducing the coefficient of dynamic friction between silicone rubber and a member brought in contact with the silicone rubber. Due to this, when silicone rubber is adapted a blood vessel model and the member brought in contact with the silicone rubber is a catheter, the resistance at the time of inserting a catheter into the blood vessel model is reduced so that an operator can smoothly insert the catheter into the blood vessel model.

On the other hand, the role of the water-soluble ion compound resides in preventing adhesion between silicone rubber and a member brought in contact with the silicone rubber (namely, reducing the coefficient of static friction) in addition to reducing the above-described coefficient of dynamic friction.

Namely, the lubricity regulating liquid according to the above example is characterized in that not only the coefficient of dynamic friction, but also the coefficient of static friction between silicone rubber and a member brought in contact with the silicone rubber is reduced by the coexistence of a surfactant and a water-soluble ion compound.

However, when the surfactant is a cationic surfactant, the adhesion between silicone rubber and a member brought in contact with the silicone rubber can be prevented even if the lubricity regulating agent does not contain the water-soluble ion compound. However, even in this case, the feeling of resistance at the time of inserting a catheter is reduced by further adding a water-soluble metal salt.

One or two or more selected from the group consisting of a cationic surfactant, an anionic surfactant, a nonionic surfactant and a zwitterionic surfactant can be used as the surfactant.

Among these surfactants, a cationic surfactant, when used together with a water-soluble ion compound, brings especially small increases in resistance at a meandering site and adhesion, and is thus preferred. Also, the cationic surfactant has excellent bactericidal action, and thus can exert the antifungal and bactericidal effects on a silicone rubber surface.

On the other hand, a nonionic surfactant is also preferably used as the surfactant. The nonionic surfactant can prevent a rise in ion concentration of the lubricity regulating liquid due to the addition of the surfactant, and makes the pH close to neutrality, so that metals in the argent is free from corrosion.

The surfactant concentration may be appropriately regulated depending, for example, on the kind of surfactant, and preferably ranges from 0.005 mmol/L or more and 100 mmol/L or less. A surfactant concentration of less than 0.005 mmol/L is not preferred as causing large contact resistance between silicone rubber and a member brought in contact with the silicone rubber and also causing adhesion. An amount of the surfactant to be incorporated which exceeds 100 mmol/L is not preferred as bringing impaired physical properties of the lubricity regulating liquid (causing slimy feeling). More preferably, the amount is 0.05 mmol/L or more and 10 mmol/L or less.

A water-soluble metal salt, a water-soluble ammonium salt (for example, ammonium chloride and ammonium sulfate) and the like can be used as the water-soluble ion compound. One or two or more selected from the group consisting of an alkali metal salt, an alkali earth metal salt, an aluminum salt and an iron salt can be used as the water-soluble metal salt. According to the inventor's test results, the use of these water-soluble metal salts in combination with a surfactant can enhance the lubricity of a surface of silicone rubber as compared with the use of a surfactant alone, and also can avoid the problem of adhesion. Although the reason for this has not been elucidated, it is inferred that these advantages might be obtained due to the matter that metal ions are coordinated with oxygen in the siloxane skeleton of silicone rubber, thereby inhibiting the adsorption of the surfactant and also inhibiting the formation of a hydrogen bond with a polar group existing in the member brought in contact with the silicone rubber. Therefore, the silicone rubber to which the lubricity regulating liquid according to the present invention is applied is not particularly limited so long as it is rubber having a siloxane skeleton in the basic skeleton.

The water-soluble alkyl metal salt includes sodium chloride, potassium chloride, cesium chloride, sodium sulfate, potassium sulfate, cesium sulfate, sodium nitrate, potassium nitrate and cesium nitrate. Also, the water-soluble alkali earth metal salt includes magnesium chloride, potassium chloride, barium chloride, magnesium nitrate, potassium nitrate and barium nitrate. Further, the aluminum salt includes aluminum chloride, aluminum sulfate and aluminum nitrate. The iron salt includes ferrous chloride, ferric chloride, ferrous sulfate, ferric sulfate, ferrous nitrate and ferric nitrate. In addition to the above exemplified ones, water-soluble alkali metal salts, alkali earth metal salts, organic acid salts of metals (for example, sodium acetate) and complexes can also be used.

The concentration of the water-soluble metal salt in the lubricity regulating liquid is preferably 1 mmol/L or more and 100 mmol/L or less. A water-soluble metal salt concentration of less than 1 mmol/L easily causes adhesion between silicone rubber and a member brought in contact with the silicone rubber. On the other hand, a water-soluble metal salt concentration of more than 100 mmol/L easily causes corrosion of a metal due to the lubricity regulating liquid. More preferably, the concentration is 2 mmol/L or more and 50 mmol/L or less.

On the other hand, when the water-soluble ion compound is an ammonium salt such as ammonium chloride or ammonium sulfate, the inventor has confirmed that the lubricity of a silicone rubber surface can be enhanced by adding the ammonium salt in an amount of 0.03 mol/L or more (preferably, 0.07 mol/L).

The lubricity regulating liquid can be prepared by adding water to a preparation comprising a surfactant and a water-soluble ion compound for dissolution.

Namely, the preparation is a preparation for a lubricity regulating liquid for regulating the lubricity of a surface of silicone rubber, the preparation comprising a surfactant and a water-soluble ion compound.

Also, the regulating kit is a kit for preparing a lubricity regulating liquid for regulating the lubricity of a surface of silicone rubber, the kit comprising a first agent comprising a surfactant and a second agent comprising a water-soluble ion compound. According to this preparation kit, the first and second agents are mixed, thereby making it possible to easily prepare a lubricity regulating liquid.

### [Brief Description of the Drawings]

FIG. 1 is a front view of a pseudo blood vessel model used in a lubricity evaluation test.
FIG. 2 is a graph showing the relation between the rotation angle and the concentration of a metal salt in a test for evaluating the lubricity of lubricity regulating liquids of Examples 1 to 8.
FIG. 3 is a graph showing the relation between the rotation angle and the concentration of a metal salt in a test for evaluating the lubricity of lubricity regulating liquids of Examples 9 and 16.
FIG. 4 is a graph showing the relation between the rotation angle and the concentration of a metal salt in a test for evaluating the lubricity of lubricity regulating liquids of Examples 10 and 11 and Comparative Examples 10 and 11.
FIG. 5 is a graph showing the relation between the rotation angle and the concentration of a metal salt in a test for evaluating the lubricity of lubricity regulating liquids of Example 12 and Comparative Example 12.
FIG. 6 is a graph showing the relation between the rotation angle and the concentration of a metal salt in a test for evaluating the lubricity of lubricity regulating liquids of Example 13 and Comparative Example 13.
FIG. 7 is a graph showing the relation between the rotation angle and the concentration of added bittern in a test for evaluating the lubricity of a lubricity regulating liquid of Example 14.
FIG. 8 is a graph showing the relation between the rotation angle and the concentration of an added surfactant in a test for evaluating the lubricity of a lubricity regulating liquid of Comparative Example 14-1.
FIG. 9 is a graph showing the relation between the rotation angle and the concentration of an added bittern in a test for evaluating the lubricity of a lubricity regulating liquid of Comparative Example 14-2.
FIG. 10 is a graph showing the relation between the rotation angle and the amount of added MgCl₂ in a lubricity evaluation test using silicone rubber (1) and silicone rubber (2).

### [Modes for Carrying out the Invention]

### [Examples]

Hereinafter, the present invention will be explained in detail by way of Examples, but is not limited to the following Examples. Various modified embodiments are also encompassed within the scope of the present invention, so long as they would be obvious to those skilled in the art without departing from the scope of claims.

### (Examples 1 to 8)

In Examples 1 to 8, a 16-wt% aqueous solution containing, as surfactants, sodium alkyl ether sulfate and fatty acid alkanol amide in a weight ratio of 2:1 was used, and various water-soluble metal salts as indicated in Table 1 were added thereto, thereby preparing lubricity regulating liquids. In the respective Examples, the water-soluble metal salt concentrations were defined as 6 types: 0.0025, 0.005, 0.01, 0.03, 0.07 and 0.20 (mol/L).

### (Comparative Examples 1 to 8)

In Comparative Examples 1 to 8, no water-soluble metal salt was added (namely, all of Comparative Examples 1 to 8 have the same composition). In the other respects, they were similar to Examples 1 to 8, and thus are not explained herein.

**[Table 1]**

| | Composition of lubricity regulating liquid | | |
|---|---|---|---|
| | Water-soluble metal salt* | Surfactant | Distilled water |
| Example 1 | NaCl | 320 mg | Distilled water 1L |
| Example 2 | KCl | 320 mg | Distilled water 1L |
| Example 3 | MgCl₂ • 6H₂O | 320 mg | Distilled water 1L |
| Example 4 | CaCl₂ • 2H₂O | 320 mg | Distilled water 1L |
| Example 5 | Al₂(SO₄)₃ • 14-18H₂O | 320 mg | Distilled water 1L |
| Example 6 | FeCl₃ • 6H₂O | 320 mg | Distilled water 1L |
| Example 7 | MgCl₂ • 6H₂O+NaCl (molar composition ratio 1:1) | 320 mg | Distilled water 1L |
| Example 8 | CH₃CO₂Na | 320 mg | Distilled water 1L |

| | | | |
|---|---|---|---|
| * The water-soluble metal salt concentrations were defined as 6 types: 0.0025, 0.005, 0.01, 0.03, 0.07 and 0.20 (mol/L). | | | |

### <Evaluation>

Tests for evaluating the lubricity and adhesiveness of the lubricity regulating liquids of Examples 1 to 8 and Comparative Examples 1 to 8 as described above to silicone rubber were conducted. The evaluating methods are as follows.

### • Lubricity evaluating test

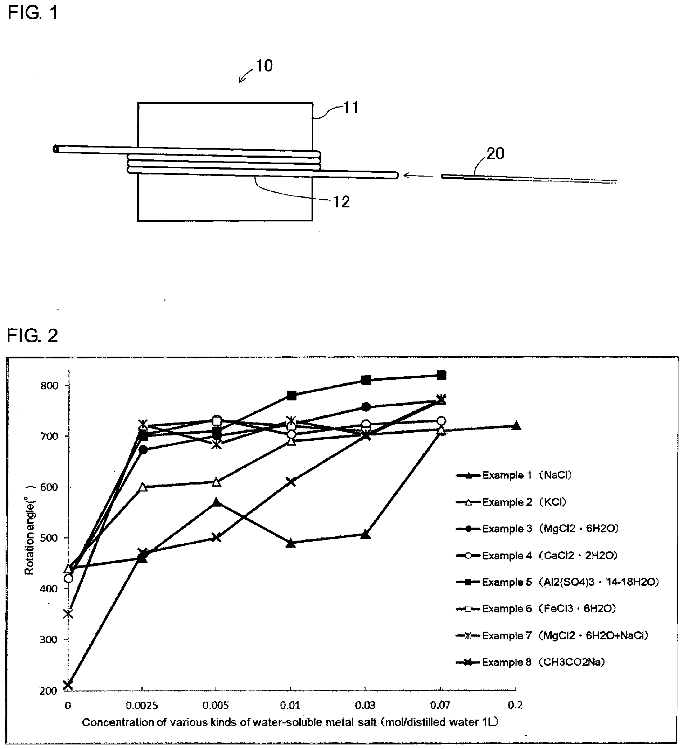

A Silicone tube (tradename: LABORAN SILICONE TUBE manufactured by AS ONE Corporation) having an internal diameter of 3 mm was provided. As shown in FIG. 1, a silicone tube 12 was wound, three times, around a cylindrical pipe 11 made of a transparent acrylic resin having a diameter of 7 cm to be fixed thereon, thereby providing a pseudo blood vessel model 10 of which both ends were protruded and extended. A catheter 20 (manufactured by Chaperone Co., thickness: 6.0 F (2.0 mm φ) was manually inserted, with constant force, from one end side of the silicone tube 12, so that the catheter 20 entered the silicone tube while rotating. Further, the catheter 20 continued to be inserted until it could not resist the frictional resistance and therefore stopped. The rotation angle at a position where the catheter 20 stopped was measured, and this angle was defined as an index of lubricity. Measurement was carried out a plurality of times.

### • Adhesiveness evaluating test

Further, the catheter 20 was retained as it was for about 10 seconds at a position where it stopped, and thereafter pulled in a direction where the catheter 20 would retract. The case where it could be easily pulled out was evaluated as "no adhesion" and the case where it could not be easily pulled out was evaluated as "adhesion." Measurement was carried out three times. Even if adhesion occurred, the catheter 20 could be pulled out by quickly repeating the application of force toward the entering direction and force toward the retracting direction to the catheter 20.

Table 2 indicates the results on the rotation angle and the presence or absence of adhesion for Examples 1 to 8 and Comparative Examples 1 to 8 as described above (the cases where the metal salt concentration is 0 in this table correspond to Comparative Examples 1 to 8). FIG. 2 indicates a graph showing the relation between the rotation angle and the metal salt concentration.

**[Table 2]**

| Example 1 | | | | | | | | | | | | | | |
|---|---|---|---|---|---|---|---|---|---|---|---|---|---|---|
| | Concentration of NaCl (mol of NaCl/1L of distilled water) | | | | | | | | | | | | | |
| | 0 | | 0.0025M | | 0.005M | | 0.01M | | 0.03M | | 0.07M | | 0.2M | |
| | Rotation angle (°) | Adhesion 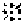 | Rotation angle (°) | Adhesion 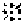 | Rotation angle (°) | Adhesion 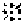 | Rotation angle (°) | Adhesion 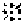 | Rotation angle (°) | Adhesion 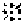 | Rotation angle (°) | Adhesion 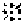 | Rotation angle (°) | Adhesion |
| 1st | 540 | - | 420 | - | 600 | + | 570 | - | 440 | - | 690 | - | 720 | - |
| 2nd | 360 | - | 420 | - | 630 | - | 450 | - | 570 | - | 720 | - | 720 | - |
| 3rd | 420 | + | 540 | - | 480 | - | 450 | - | 510 | - | 720 | - | 720 | - |
| Average | 440 | | 460 | | 570 | | 490 | | 507 | | 710 | | 720 | |
| | | | | | | | | | | | | | | |

| Example 2 | | | | | | | | | | | | | | |
|---|---|---|---|---|---|---|---|---|---|---|---|---|---|---|
| | Concentration of KCl (mol of KCl/1L of distilled water) | | | | | | | | | | | | | |
| | 0 | | 0.0025M | | 0.005M | | 0.01M | | 0.03M | | 0.07M | | | |
| | Rotation angle (°) | Adhesion 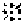 | Rotation angle (°) | Adhesion 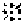 | Rotation angle (°) | Adhesion 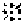 | Rotation angle (°) | Adhesion 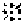 | Rotation angle (°) | Adhesion 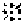 | Rotation angle (°) | Adhesion 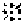 | | |
| 1st | 420 | - | 540 | - | 510 | - | 690 | - | 690 | - | 730 | - | | |
| 2nd | 510 | - | 630 | - | 630 | - | 710 | - | 710 | - | 690 | - | | |
| 3rd | 390 | - | 630 | - | 690 | - | 670 | - | 710 | - | 720 | - | | |
| Average | 440 | | 600 | | 610 | | 690 | | 703 | | 713 | | | |
| | | | | | | | | | | | | | | |

| Example 3 | | | | | | | | | | | | | | |
|---|---|---|---|---|---|---|---|---|---|---|---|---|---|---|
| | Concentration of MgCl₂ (mol of MgChl₂/1L of distilled water) | | | | | | | | | | | | | |
| | 0 | | 0.0025M | | 0.005M | | 0.01M | | 0.03M | | 0.07M | | | |
| | Rotation angle (°) | Adhesion 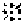 | Rotation angle (°) | Adhesion 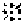 | Rotation angle (°) | Adhesion 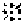 | Rotation angle (°) | Adhesion 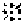 | Rotation angle (°) | Adhesion 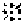 | Rotation angle (°) | Adhesion 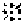 | | |
| 1st | 460 | + | 690 | - | 710 | - | 700 | - | 810 | - | 810 | - | | |
| 2nd | 410 | - | 660 | - | 700 | - | 720 | - | 730 | - | 750 | - | | |
| 3rd | 390 | - | 670 | - | 690 | - | 750 | - | 730 | - | 750 | - | | |
| Average | 420 | | 673 | | 700 | | 723 | | 757 | | 770 | | | |
| | | | | | | | | | | | | | | |

| Example 4 | | | | | | | | | | | | | | |
|---|---|---|---|---|---|---|---|---|---|---|---|---|---|---|
| | Concentration of CaCl₂ (mol of CaCl₂/1L of distilled water) | | | | | | | | | | | | | |
| | 0 | | 0.0025M | | 0.005M | | 0.01M | | 0.03M | | 0.07M | | | |
| | Rotation angle (°) | Adhesion 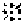 | Rotation angle (°) | Adhesion 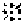 | Rotation angle (°) | Adhesion 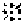 | Rotation angle (°) | Adhesion 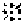 | Rotation angle (°) | Adhesion 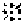 | Rotation angle (°) | Adhesion 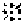 | | |
| 1st | 420 | - | 690 | - | 700 | - | 720 | - | 720 | - | 780 | - | | |
| 2nd | 390 | - | 720 | - | 780 | - | 690 | - | 720 | - | 720 | - | | |
| 3rd | 450 | - | 700 | - | 720 | - | 700 | - | 730 | - | 690 | - | | |
| Average | 420 | | 703 | | 733 | | 703 | | 723 | | 730 | | | |
| | | | | | | | | | | | | | | |

| Example 5 | | | | | | | | | | | | | | |
|---|---|---|---|---|---|---|---|---|---|---|---|---|---|---|
| | Concentration of Al₂(SO₄)₃ (mol of Al₂(SO₄)₃/1L of distilled water) | | | | | | | | | | | | | |
| | 0 | | 0.0025M | | 0.005M | | 0.01M | | 0.03M | | 0.07M | | | |
| | Rotation angle (°) | Adhesion 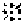 | Rotation angle (°) | Adhesion 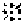 | Rotation angle (°) | Adhesion 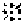 | Rotation angle (°) | Adhesion 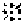 | Rotation angle (°) | Adhesion 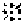 | Rotation angle (°) | Adhesion 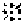 | | |
| 1st | 360 | + | 690 | - | 720 | - | 780 | - | 810 | - | 810 | - | | |
| 2nd | 360 | - | 690 | - | 690 | - | 780 | - | 810 | - | 810 | - | | |
| 3rd | 390 | - | 720 | - | 720 | - | 780 | - | 810 | - | 840 | - | | |
| Average | | | 700 | | 710 | | 780 | | 810 | | 820 | | | |
| | | | | | | | | | | | | | | |

| Example 6 | | | | | | | | | | | | | | |
|---|---|---|---|---|---|---|---|---|---|---|---|---|---|---|
| | Concentration of FeCl₃ (mol of FeCl₃/1L of distilled water) | | | | | | | | | | | | | |
| | 0 | | 0.0025M | | 0.005 M | | 0.01M | | 0.03M | | | | | |
| | Rotation angle (°) | Adhesion | Rotation angle (°) | Adhesion | Rotation angle (°) | Adhesion | Rotation angle (°) | Adhesion | Rotation angle (°) | Adhesion | | | | |
| 1st | 390 | - | 720 | - | 750 | - | 750 | - | 720 | - | | | | |
| 2nd | 330 | + | 720 | - | 720 | - | 720 | - | 720 | - | | | | |
| 3rd | 450 | + | 720 | - | 720 | - | 690 | - | 690 | - | | | | |
| Average | 390 | | 720 | | 730 | | 720 | | 710 | | | | | |
| | | | | | | | | | | | | | | |

| Example 7 | | | | | | | | | | | | | | |
|---|---|---|---|---|---|---|---|---|---|---|---|---|---|---|
| | Concentration of MgCl₂ and NaCl (mol of MgCl₂ and NaCl/1L of distilled water) (molar number of MgCl₂ : molar number of NaCl = 1:1) | | | | | | | | | | | | | |
| | 0 | | 0.0025M | | 0.005 M | | 0.01M | | 0.03M | | 0.07M | | | |
| | Rotation angle (°) | Adhesion 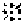 | Rotation angle (°) | Adhesion 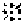 | Rotation angle (°) | Adhesion 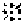 | Rotation angle (°) | Adhesion 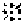 | Rotation angle (°) | Adhesion 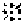 | Rotation angle (°) | Adhesion 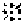 | | |
| 1st | 320 | - | 690 | - | 670 | - | 750 | - | 690 | - | 780 | - | | |
| 2nd | 370 | - | 700 | - | 690 | - | 720 | - | 700 | - | 780 | - | | |
| 3rd | 360 | - | 780 | - | 690 | - | 720 | - | 720 | - | 760 | - | | |
| Average | 350 | | 723 | | 683 | | 730 | | 703 | | 773 | | | |
| | | | | | | | | | | | | | | |

| Example 8 | | | | | | | | | | | | | | |
|---|---|---|---|---|---|---|---|---|---|---|---|---|---|---|
| | Concentration of CH₃CO₂Na (mol of CH₃CO₂Na /1 L of distilled water) | | | | | | | | | | | | | |
| | 0 | | 0.0025M | | 0.005M | | 0.01M | | 0.03M | | 0.07M | | 0.10M | |
| | Rotation angle (°) | Adhesion 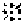 | Rotation angle (°) | Adhesion 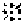 | Rotation angle (°) | Adhesion 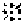 | Rotation angle (°) | Adhesion 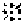 | Rotation angle (°) | Adhesion 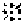 | Rotation angle (°) | Adhesion 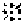 | Rotation angle (°) | Adhesion 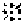 |
| 1st | 210 | - | 480 | - | 480 | - | 570 | - | 690 | - | 720 | - | 870 | - |
| 2nd | 210 | - | 450 | - | 510 | - | 600 | - | 690 | - | 780 | - | 780 | - |
| 3rd | 210 | - | 480 | - | 510 | - | 660 | - | 720 | - | 810 | - | 810 | - |
| Average | 210 | | 470 | | 500 | | 610 | | 700 | | 770 | | 820 | |

| | | | | | | | | | | | | | | |
|---|---|---|---|---|---|---|---|---|---|---|---|---|---|---|
| * [+] ... Adhesion occurred. [-] ... No adhesion occurred. [±] ... Judgement could not be made. | | | | | | | | | | | | | | |

As shown in FIG. 2, in Examples 1 to 8, the rotation angle was drastically raised by adding the water-soluble metal salts even though the surfactant concentration was constant. The rotation angle rose as the amount of the water-soluble metal salts (NaCl, KCl, MgCl₂, CaCl₂, Al₂(SO₃), FeCl₃, MgCl₂ + NaCl, CH₃COONa) to be added was increased, and became an almost constant value at a water-soluble metal salt concentration of 0.005 mol/L or more. From the above results, it has been found that the lubricity of the silicone rubber surface drastically improves due to the use of the surfactant and the water-soluble metal salt in combination.

Also, as indicated in Table 2, adhesion occurred in some cases (adhesion occurred four times in twenty four measurements) in the case where no water-soluble metal salt was added to the surfactant (i.e., in Comparative Examples 1 to 8), whereas no adhesion occurred in the case where the water-soluble metal salt was added in an amount of 0.0025 mol/L or more.

### (Examples 9 to 13)

In Examples 9 to 13, the water-soluble metal salt used was magnesium chloride in all the cases, and it was mixed with various surfactants to prepare lubricity regulating liquids (see Table 3). The surfactant concentrations of the prepared solutions were defined as 0, 0.00005, 0.000075, 0.0001, 0.0002, 0.0004, 0.0005, 0.00075, 0.001, 0.002 and 0.004 (mol/L) for the respective Examples.

### (Example 16 and Comparative Examples 10 to 13)

In Example 16 and Comparative Examples 10 to 13, no magnesium chloride was added, and they were similar to Examples 9 to 13 in the other respects (see Table 3).

**[Table 3]**

| | Lubricity regulating liquid | | |
|---|---|---|---|
| | Distilled water | Surfactant | MgCl₂ • 6H₂O |
| Example 9 | Distilled water 1L | Trimethylstearylammonium chloride (cationic) | 0.61 g |
| Example 16 | Distilled water 1L | Trimethylstearylammonium chloride (cationic) | Not added |
| Example 10 | Distilled water 1L | Sodium lauryl sulfate (anionic) | 0.61 g |
| Comparative Example 10 | Distilled water 1L | Sodium lauryl sulfate (anionic) | Not added |
| Example 11 | Distilled water 1L | Sodium 1-dodecanesulfonate (anionic) | 0.61 g |
| Comparative Example 11 | Distilled water 1L | Sodium 1-dodecanesulfonate (anionic) | Not added |
| Example 12 | Distilled water 1L | Myristyl sulfobetaine (zwitterionic) | 0.61 g |
| Comparative Example 12 | Distilled water 1L | Myristyl sulfobetaine (zwitterionic) | Not added |
| Example 13 | Distilled water 1L | Tween 20 (nonionic) | 0.61 g |
| Comparative Example 13 | Distilled water 1L | Tween 20 (nonionic) | Not added |

### <Evaluation>

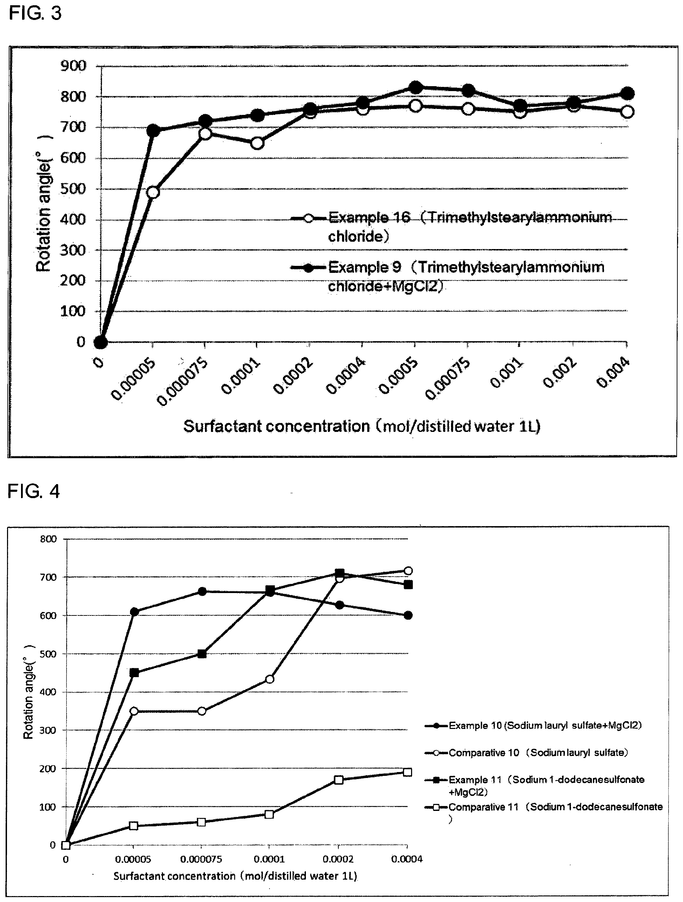

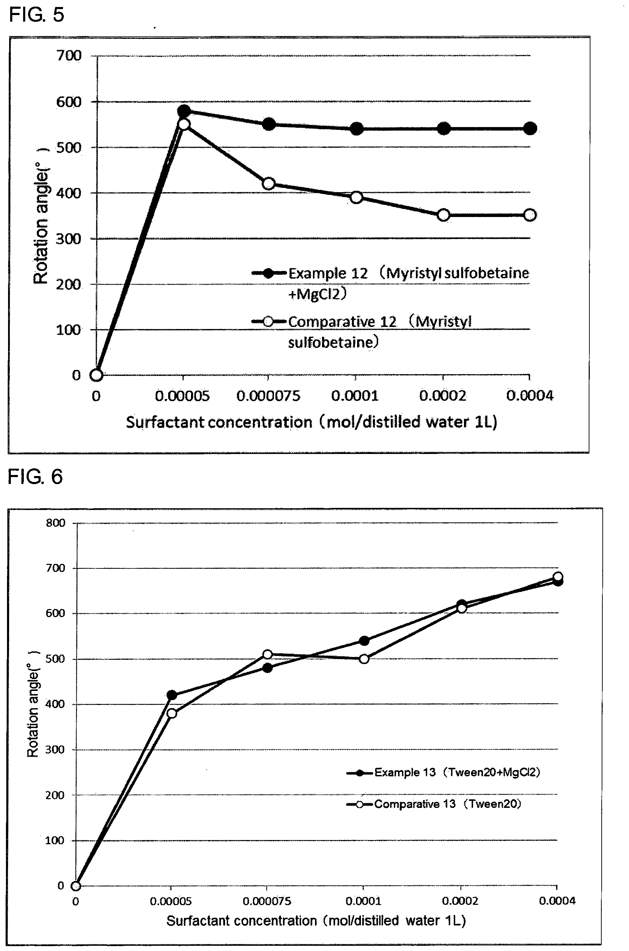

For the lubricity regulating liquids of Examples 9 to 13, Example 16 and Comparative Examples 10 to 13, the evaluation results on lubricity are shown in FIGs. 3 and 6, and those of adhesiveness are shown in Table 4. In the meantime, a LABORAN SILICONE TUBE having an internal diameter of 3 mm (manufactured by AS ONE Corporation) was employed as the silicone tube used in the evaluation of lubricity.

**[Table 4]**

| | Concentration of surfactant (mmol/L) | | | | | | | | | |
|---|---|---|---|---|---|---|---|---|---|---|
| | 0 | 0.05 | 0.075 | 0.1 | 0.2 | 0.4 | 0.75 | 1 | 2 | 4 |
| Example 9* | + | - | - | - | - | - | - | - | - | - |
| Example 16* | + | - | - | - | - | - | - | - | - | - |
| Example 10 | + | - | - | - | - | - | | | | |
| Comparative Example 10 | + | + | - | - | - | - | | | | |
| Example 11 | + | - | - | - | - | - | | | | |
| Comparative Example 11 | + | + | + | + | + | - | | | | |
| Example 12 | + | - | - | - | - | - | | | | |
| Comparative Example 12 | + | - | - | - | - | - | | | | |
| Example 13* | + | - | - | - | - | - | | | | |
| Comparative Example 13 * | + | + | - | - | - | - | | | | |

| | | | | | | | | | | |
|---|---|---|---|---|---|---|---|---|---|---|
| "+" Adhesion "-" No adhesion * The catheter insertion resistance is Example 9 < Example 16, Example 13 < Comparative Example 13. | | | | | | | | | | |

In Examples 9 and 16 using a cationic surfactant trimethylstearylammonium chloride, Example 9 in which magnesium chloride was added was superior to Example 16 in which no magnesium chloride was added in terms of the lubricity at a low surfactant concentration of 0.2 mmol/L or less, as shown in FIG. 3. As regards adhesiveness, no adhesion occurred at a surfactant concentration of 0.05 mmol/L or more in either Examples 9 and 16. However, the feeling of resistance at the time of catheter insertion in Example 9 was apparently smaller than that in Example 16, and it has been found that the coexistence of magnesium chloride and a cationic surfactant enabled quite smooth insertion of a catheter.

In Examples 10 and 11 and Comparative Examples 10 and 11 using an anionic surfactant, Examples 10 and 11 in which magnesium chloride was added were superior, in terms of lubricity, to Comparative Examples 10 and 11 in which no magnesium chloride was added, as shown in FIG. 4. As regards adhesiveness, no adhesion occurred at a surfactant concentration of 0.05 mmol/L or more in Examples 10 and 11, whereas adhesion occurred at a surfactant concentration of 0.05 mmol/L or less in Comparative Example 10 and at a surfactant concentration of 0.2 mmol/L or less in Comparative Example 11. In view of the above, it has been found that the coexistence of an anionic surfactant and magnesium chloride improved both lubricity and non-adhesiveness.

Further, in Example 12 and Comparative Example 12 using a zwitterionic (betaine-type) surfactant, Example 12 in which magnesium chloride was added was superior, in terms of lubricity, to Comparative Example 12 in which no magnesium chloride was added, as shown in FIG. 5. As regards adhesiveness, no adhesion occurred at a surfactant concentration of 0.05 mmol/L or more in Example 12 and Comparative Example 12, as indicated in Table 4 above. In view of the above, it has been found that the coexistence of a zwitterionic (betaine type) surfactant and magnesium chloride improved both lubricity and non-adhesiveness.

Also, in Example 13 and Comparative Example 13 using a nonionic surfactant, both Example 13 and Comparative Example 13 exhibited excellent lubricity.

As regards adhesiveness, adhesion occurred at a surfactant concentration of 0.05 mmol/L or less in Comparative Example 13, whereas no adhesion occurred at a surfactant concentration of 0.05 mmol/L or more in Example 13, as indicated in Table 4 above. Further, the feeling of resistance at the time of catheter insertion in Example 13 was apparently smaller than that in Comparative Example 13, and it has been found that the coexistence of magnesium chloride and a nonionic surfactant enabled quite smooth insertion of a catheter.

### (Example 14)

In Example 14, prepared was a solution mixture containing 500 ml of distilled water, 1 ml of a commercial kitchen detergent (mixture of anionic surfactant + nonionic surfactant: concentration: 16% by weight) and a commercially-available bittern solution (Aranami no Honnigari, manufactured by AKO ARANAMI SHIO CO., LTD.; 100 ml of this bittern solution contains 4318 mg of magnesium, 3810 mg of potassium, 3048 mg of sodium and 2032 mg of calcium). This solution mixture was used as a lubricity regulating liquid. The bittern concentrations were defined as 0, 0.25, 5, 10 and 15 (ml per liter of distilled water). The lubricity and adhesiveness were respectively evaluated by the methods for evaluating lubricity and adhesiveness as described above. In the meantime, a LABORAN SILICONE TUBE having an internal diameter of 3 mm (manufactured by AS ONE Corporation) was employed as the silicone tube used in the evaluation of lubricity.

### (Comparative Example 14-1)

In Comparative Example 14-1, a liquid comprising no bittern but similar to that prepared in Example 14 in the other respects was employed as the lubricity regulating liquid.

### (Comparative Example 14-2)

In Comparative Example 14-2, a liquid comprising no surfactant but similar to that prepared in Example 14 in the other respects was employed as the lubricity regulating liquid.

### <Evaluation>

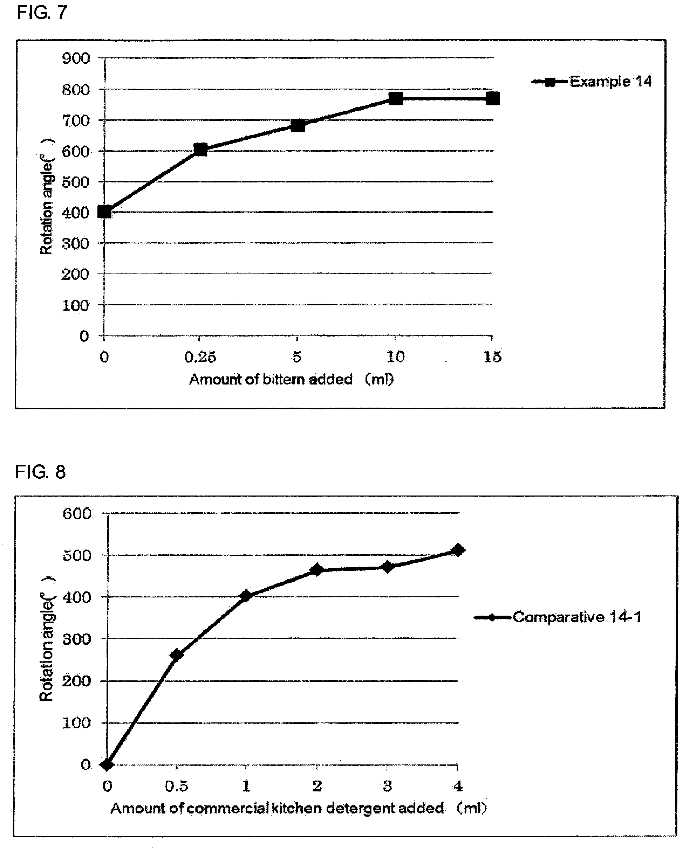

The evaluations of lubricity and adhesiveness for Example 14, Comparative Example 14-1 and Comparative Example 14-2 are indicated in Tables 5 to 7. The graphs concerning lubricity are shown in FIGs. 7 to 9. In the meantime, a LABORAN SILICONE TUBE having an internal diameter of 3 mm (manufactured by AS ONE Corporation) was employed as the silicone tube used in the evaluation of lubricity.

**[Table 5]**

| Various concentrations of bittern + 1 ml of commercially-available kitchen detergent + 500 ml of distilled water. | | | | | | | | | | |
|---|---|---|---|---|---|---|---|---|---|---|
| | Amount of bittern added (ml) | | | | | | | | | |
| | 0 | | 0.25 ml | | 5 ml | | 10 ml | | 15 ml | |
| | Rotation angle (°) | Adhesion* | Rotation angle (°) | Adhesion* | Rotation angle (°) | Adhesion* | Rotation angle (°) | Adhesion* | Rotation angle (°) | Adhesion* |
| 1st | 390 | - | 510 | - | 600 | - | 780 | - | 780 | - |
| 2nd | 420 | - | 540 | - | 660 | - | 810 | - | 810 | - |
| 3rd | 600 | - | 510 | - | 660 | - | 780 | - | 810 | - |
| 4th | 360 | - | 630 | - | 690 | - | 810 | - | 810 | - |
| 5th | 360 | - | 630 | - | 720 | - | 810 | - | 810 | - |
| 6th | 360 | + | 680 | - | 690 | - | 810 | - | 780 | - |
| 7th | 420 | - | 690 | - | 720 | - | 750 | - | 840 | - |
| 8th | 400 | + | 630 | - | 720 | - | 720 | - | 750 | - |
| 9th | 400 | - | 600 | - | 690 | - | 720 | - | 810 | - |
| 10th | 360 | + | 540 | - | 690 | - | 750 | - | 630 | - |
| 11th | 390 | + | 660 | - | 690 | - | 770 | - | 660 | - |
| 12th | 360 | + | 630 | - | 670 | - | 700 | - | 720 | - |
| Average | 402 | | 604 | | 683 | | 768 | | 768 | |

| | | | | | | | | | | |
|---|---|---|---|---|---|---|---|---|---|---|
| "'+" Adhesion occurred. "-" No adhesion occurred. | | | | | | | | | | |

**[Table 6]**

| Various concentrations of commercially-available kitchen detergent + 500 ml of distilled water. 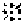 | | | | | | | | | | | | |
|---|---|---|---|---|---|---|---|---|---|---|---|---|
| | Amount of commercial kitchen detergent added (ml) | | | | | | | | | | | |
| | 0 | | 0.5 ml | | 1 ml | | 2 ml | | 3 ml | | 4 ml | |
| | Rotation angle (°) | Adhesion 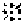 | Rotation angle (°) | Adhesion 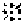 | Rotation angle (°) | Adhesion 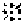 | Rotation angle (°) | Adhesion 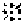 | Rotation angle (°) | Adhesion 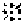 | Rotation angle (°) | Adhesion 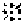 |
| 1st | 0 | + | 180 | + | 390 | - | 480 | - | 480 | - | 450 | - |
| 2nd | 0 | + | 240 | + | 420 | - | 450 | - | 600 | - | 600 | - |
| 3rd | 0 | + | 240 | + | 600 | - | 600 | - | 660 | - | 690 | - |
| 4th | 0 | + | 270 | + | 360 | - | 360 | - | 450 | - | 510 | - |
| 5th | 0 | + | 330 | + | 360 | - | 420 | - | 420 | - | 690 | - |
| 6th | 0 | + | 330 | + | 360 | + | 450 | - | 480 | - | 570 | - |
| 7th | 0 | + | 240 | + | 420 | - | 510 | - | 510 | - | 450 | - |
| 8th | 0 | + | 210 | + | 400 | + | 510 | - | 510 | - | 450 | - |
| 9th | 0 | + | 270 | + | 400 | - | 510 | + | 400 | + | 450 | - |
| 10th | 0 | + | 240 | + | 360 | + | 400 | + | 400 | - | 400 | - |
| 11th | 0 | + | 270 | - | 390 | + | 480 | - | 350 | - | 450 | - |
| 12th | 0 | + | 300 | - | 360 | + | 400 | - | 390 | - | 420 | - |
| Average | 0 | | 260 | | 402 | | 464 | | 471 | | 511 | |

| | | | | | | | | | | | | |
|---|---|---|---|---|---|---|---|---|---|---|---|---|
| *[+] ... Adhesion occurred. [-] ... No adhesion occurred. [±] ... Judgement could not be made. | | | | | | | | | | | | |

**[Table 7]**

| 500 ml of Distilled water + bittern. | | | | | | | | | | |
|---|---|---|---|---|---|---|---|---|---|---|
| | Amount of bittern added (ml) | | | | | | | | | |
| | 0 | | 0.25 ml | | 5 ml | | 10 ml | | 15 ml | |
| | Rotation angle (°) | Adhesion 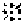 | Rotation angle (°) | Adhesion 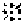 | Rotation angle (°) | Adhesion 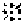 | Rotation angle (°) | Adhesion 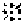 | Rotation angle (°) | Adhesion 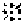 |
| 1st | 0 | + | 30 | - | 60 | - | 30 | - | 30 | - |
| 2nd | 0 | + | 60 | - | 60 | - | 30 | - | 60 | - |
| 3rd | 0 | + | 30 | - | 60 | - | 30 | + | 30 | - |
| 4th | 0 | + | 30 | + | 30 | - | 30 | - | 40 | + |
| 5th | 0 | + | 30 | + | 30 | - | 30 | + | 40 | + |
| 6th | 0 | + | 50 | + | 30 | - | 20 | - | 40 | + |
| 7th | 0 | + | 40 | + | -20 | - | -20 | | 30 | |
| 8th | 0 | + | -10 | + | 0 | - | -30 | | 20 | |
| 9th | 0 | + | 30 | + | 30 | + | -10 | | 10 | |
| 10th | 0 | + | 30 | - | 40 | + | -30 | - | 30 | - |
| 11th | 0 | + | 40 | + | 0 | - | -10 | - | 40 | + |
| 12th | 0 | + | 30 | - | -20 | + | -10 | - | 30 | - |
| Average | 0 | | 33 | | 25 | | 5 | | 33 | |

| | | | | | | | | | | |
|---|---|---|---|---|---|---|---|---|---|---|
| "'+" Adhesion occurred. "-" No adhesion occurred. | | | | | | | | | | |

### • Evaluation results on lubricity

As shown in FIG. 7 and Table 5, in Example 14, the addition of bittern to the solution mixture of distilled water and a commercially-available kitchen detergent as the lubricity regulating liquid drastically raised the rotation angle as compared with that before addition. Further, the rotation angle rose as the amount of the bittern added was increased, and became a constant value when the bittern concentration reached a certain level. The maximum value of the rotation angle was 768°.

In Comparative Example 14-1 in which no bittern was added, as shown in FIG. 8 and Table 6, the rotation angle rose as the amount of the commercially-available kitchen detergent added was increased, and became a constant value when the commercially-available kitchen detergent concentration reached a certain level. However, the maximum value of the rotation angle was 511°, which was significantly lower than the maximum value 768° for the rotation angle in Example 14 in which bittern was added to the solution mixture.

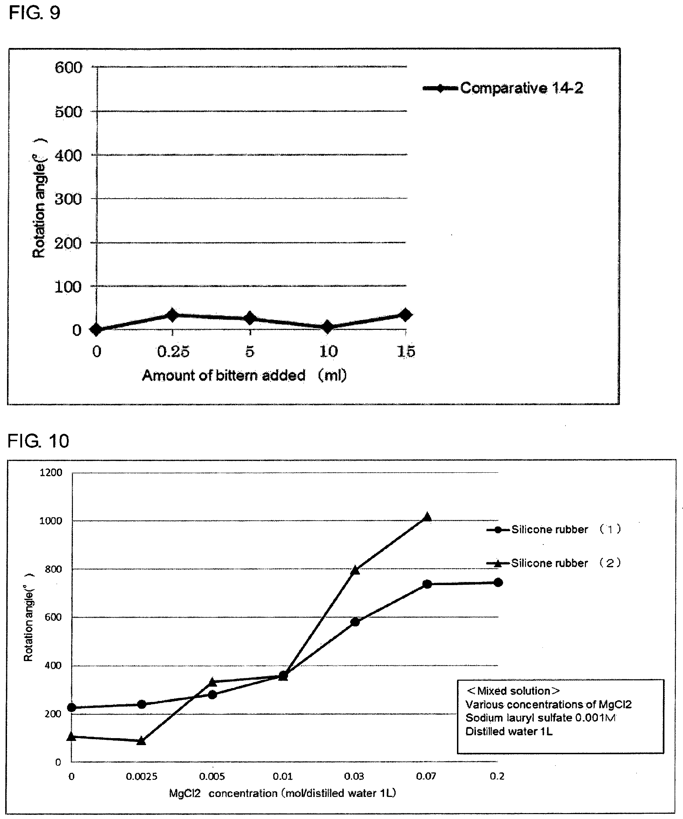

Further, in Comparative Example 14-2 in which no surfactant was added, as shown in FIG. 9 and Table 7, lubricity was quite poor so that the catheter could hardly be inserted.

### • Evaluation results on adhesiveness

In Example 14, no adhesion occurred as indicated in Table 5. On the other hand, in Comparative Example 14-1, adhesion occurred when the surfactant concentration was not a certain value or more as indicated in Table 6. Further, in Comparative Example 14-2, adhesion occurred in some cases even when the bittern concentration was increased as indicated in Table 7.

From the above results, it has been found that a lubricity regulating liquid containing a surfactant and bittern in combination could improve the lubricity of a silicone rubber surface and prevent an adhesion phenomenon.

### [Evaluation of various kinds of silicone rubber]

As Example 15, there was prepared a lubricity regulating liquid consisting of a solution mixture of 1 L of distilled water, 0.29 g of sodium lauryl sulfate (0.001 mol/L) and magnesium chloride (hexahydrate). The concentrations of magnesium chloride in the lubricity regulating liquid were defined as 0, 0.0025, 0.005, 0.01, 0.03, 0.07 and 0.20 (mol per liter of distilled water).

The lubricity regulating liquid of Example 15 was used to evaluate the lubricity and adhesiveness of the following two kinds of silicone rubber.
Silicone rubber (1): LABORAN SILICONE TUBE having an internal diameter of 3 mm (AS ONE Corporation)
Silicone rubber (2): silicone tube obtained by coating an inner wall of a LABORAN SILICONE TUBE having an internal diameter of 6 mm (AS ONE Corporation) with ELASTOSILM8520 (manufactured by WACKER ASAHIKASEI SILICONE CO., LTD.) at a thickness of 1.5 mm

### (Evaluation)

The evaluations of lubricity and adhesiveness for silicone rubber (1) and silicone rubber (2) are indicated in Table 8. The evaluation of lubricity is shown in FIG. 10.

**[Table 8]**

| Silicone rubber (1) | | | | | | | | | | | | | | | | |
|---|---|---|---|---|---|---|---|---|---|---|---|---|---|---|---|---|
| | Only distilled water (no sodium lauryl sulfate or MgCl₂) | | Concentration of MgCl₂•6H₂O (mol of MgCl₂•6H₂O/1L of distilled water) | | | | | | | | | | | | | |
| | | | 0 | | 0.0025M | | 0.005M | | 0.01M | | 0.03M | | 0.07M | | 0.20M | |
| | Rotation angle (°) | Adhesion* | Rotation angle (°) | Adhesion* | Rotation angle (°) | Adhesion* | Rotation angle (°) | Adhesion* | Rotation angle (°) | Adhesion* | Rotation angle (°) | Adhesion* | Rotation angle (°) | Adhesion* | Rotation angle (°) | Adhesion* |
| 1st | 90 | + | 220 | + | 210 | + | 270 | - | 420 | - | 560 | - | 720 | - | 690 | - |
| 2nd | 90 | + | 240 | + | 260 | + | 290 | - | 330 | - | 560 | - | 720 | - | 750 | - |
| 3rd | 80 | + | 220 | + | 250 | + | 280 | + | 330 | - | 620 | - | 770 | - | 790 | - |
| Average | 87 | | 227 | | 240 | | 280 | | 360 | | 580 | | 737 | | 743 | |

| Silicone rubber (2) | | | | | | | | | | | | | | | | |
|---|---|---|---|---|---|---|---|---|---|---|---|---|---|---|---|---|
| | Only distilled sodium lauryl sulfate or MgCl₂) | | Concentration of MgCl₂•6H₂O (mol of MgCl₂•6H₂O/1Lof distilled water) | | | | | | | | | | | | | |
| | | | 0 | | 0.0025M | | 0.005M | | 0.01M | | 0.03M | | 0.07M | | | |
| | Rotation angle (°) | Adhesion* | Rotation angle (°) | Adhesion* | Rotation angle (°) | Adhesion* | Rotation angle (°) | Adhesion* | Rotation angle (°) | Adhesion* | Rotation angle (°) | Adhesion * | Rotation angle (°) | Adhesion* | | |
| 1st | 30 | ± | 120 | - | 60 | - | 330 | + | 410 | - | 770 | - | 990 | - | | |
| 2nd | 30 | ± | 100 | - | 90 | - | 360 | ± | 330 | - | 810 | - | 1040 | - | | |
| 3rd | 30 | ± | 100 | - | 120- | - | 310 | - | 330 | - | 810 | - | 1020 | - | | |
| Average | 30 | | 107 | | 90 | | 333 | | 357 | | 797 | | 1017 | | | |

| | | | | | | | | | | | | | | | | |
|---|---|---|---|---|---|---|---|---|---|---|---|---|---|---|---|---|
| *[+] ... Adhesion occurred. [-] ... No adhesion occurred. [±] ... Judgement could not be made. | | | | | | | | | | | | | | | | |

### • Evaluation results on lubricity

As indicated in Table 8 and FIG. 10, it has been found that, as regards both silicone rubber (1) and silicone rubber (2), the rotation angle, i.e., lubricity, was drastically increased as the amount of magnesium chloride added was increased. In view of the above, the effectiveness of silicone rubber for lubricity regardless of its kind has been strongly supported.

### • Evaluation results on adhesiveness

As indicated in Table 8, it has been found that, as regards both of silicone rubber (1) and silicone rubber (2), no adhesion phenomenon occurred as the amount of magnesium chloride added was increased. In view of the above, the effectiveness of silicone rubber also for adhesion phenomenon regardless of its kind has been strongly supported.

As indicated in the evaluation results on silicone rubber (1) and silicone rubber (2) described above, the silicone rubber to be applied to the lubricity regulating liquid is not particularly limited so long as it is rubber having a siloxane skeleton in the basic skeleton. Specific examples of such silicone rubber include:
ELASTOSIL N 2010, ELASTOSIL N 2034, ELASTOSIL N 2189, ELASTOSIL N 2197, ELASTOSIL N 9132S, ELASTOSIL RT K, WACKER SilGel 612, ELASTOSIL RT 601, ELASTOSIL RT 602, ELASTOSIL RT 604, ELASTOSIL RT 607, ELASTOSIL RT 741, ELASTOSIL RT 745, ELASTOSIL RT 745 "S," ELASTOSIL RT 707 W, ELASTOSIL RT 713, SEMICOSIL 987 GR, SEMICOSIL 988/1K, SEMICOSIL 989/1K and ELASTOSIL M8520 (all manufactured by WACKER ASAHIKASEI SILICONE CO., LTD.); and
LABORAN SILICONE TUBE (manufactured by AS ONE Corporation).

The reagents used in Examples and Comparative Examples are listed below.
- Sodium chloride (99% or more; manufactured by The Salt Industry Center of Japan)
- Potassium chloride (99% or more; manufactured by Naitou Shouten Co., Ltd.)
- Magnesium chloride-hexahydrate (99% or more; manufactured by Naitou Shouten Co., Ltd.)
- Calcium chloride (manufactured by Naitou Shouten Co., Ltd.)
- Aluminum sulfate tetradecahydrate-octadecahydrate (manufactured by Wako Pure Chemical Industries Ltd.)
- Ferric chloride-hexahydrate (manufactured by Wako Pure Chemical Industries Ltd.)
- Trimethylstearylammonium chloride (manufactured by Tokyo Chemical Industry Co., Ltd.)
- Sodium lauryl sulfate (manufactured by Tokyo Chemical Industry Co., Ltd.)
- Sodium 1-dodecanesulfonate (manufactured by Tokyo Chemical Industry Co., Ltd.)
- Myristyl sulfobetaine (manufactured by Tokyo Chemical Industry Co., Ltd.)
- Tween 20 (manufactured by Tokyo Chemical Industry Co., Ltd.)
- Bittern (Aranami no Honnigari manufactured by AKO ARANAMI SHIO CO., LTD.)

<Applicationto blood vessel model made of silicone rubber>

A blood vessel model made of silicone rubber is incorporated into a case for a human model provided by the Applicant, and a tank, piping and a pump are attached thereto as auxiliary devices. Into the tank, 101 of tap water was put, and 300 ml of a bittern solution was further injected. One hundred (100) ml of this bittern solution contains 4318 mg of magnesium, 3810 mg of potassium, 3048 mg of sodium and 2032 mg of calcium. To a reference liquid obtained by mixing a predetermined amount of bittern to water, 40 ml of a surfactant used in Example 1 described above is added. The amount of a surfactant to be added can be preferably regulated by users depending on their preference.

A pigment, a bactericide, a preservative and other aids can be added to a circulating liquid according to need. When such a circulating liquid is used, the evaluation that the feeling of insertion of a catheter into the blood vessel model is close to that at the time of an operation was obtained from a plurality of doctors.

More specifically, the catheter could be inserted, without resistance, even into a meandering portion of the blood vessel model, and no adhesion occurred therebetween even when the catheter was inserted into the blood vessel model and left as it was.

### [Explanation of Reference Numerals]

10. Pseudo blood vessel model
11. Cylindrical pipe
12. Silicone tube
• 20. Catheter

## Claims

1. Use of a lubricity regulating liquid for regulating the lubricity of a surface of silicone rubber, said lubricity regulating liquid being used as circulating liquid to be filled in a blood vessel model for a catheter simulator made of silicone rubber, said lubricity regulating liquid comprising:
water;
a cationic surfactant and/or a nonionic surfactant and/or a zwitterionic surfactant; and
a water-soluble ion compound being one or two or more selected from the group consisting of an alkali metal salt, an alkali earth metal salt, an aluminum salt and an iron salt;
wherein a concentration of the surfactant(s) ranges from 0.005 mmol/L or more to 100 mmol/L or less; and a concentration of the ion compound ranges from 1 mmol/L or more to 100 mmol/L or less.

2. Use according to claim 1, wherein the surfactant comprises at least a cationic surfactant.

## Patentansprüche

1. Verwendung einer Flüssigkeit zur Regulierung der Schlüpfrigkeit, um die Schlüpfrigkeit einer Silikongummioberfläche zu regulieren, die genannte Flüssigkeit zur Regulierung der Schlüpfrigkeit wird als zirkulierende Flüssigkeit verwendet, die in das Modell eines Blutgefäßes für einen Kathetersimulator eingeführt wird, der aus Silikongummi besteht, die genannte Flüssigkeit zur Regulierung der Schlüpfrigkeit enthält:
Wasser;
einen kationischen grenzflächenaktiven Stoff und/oder einen nicht ionischen grenzflächenaktiven Stoff und/oder einen zwitterionischen grenzflächenaktiven Stoff; und
eine oder zwei oder mehrere von der Gruppe gewählte wasserlösliche Ionenverbindung/en, welche aus einem Alkalimetallsalz, einem Erdalkalimetallsalz, einem Aluminiumsalz und einem Eisensalz besteht/bestehen;
deren Konzentration der grenzflächenaktiven Stoffe von 0.005 mmol/l oder mehr bis 100 mmol/l oder weniger variiert; und die Konzentration der Ionenverbindung von 1 mmol/l oder mehr bis 100 mmol/L oder weniger variiert.

2. Verwendung gemäß Patentanspruch 1, in dem der grenzflächenaktive Stoff mindestens einen kationischen grenzflächenaktiven Stoff enthält.

## Revendications

1. Utilisation d'un liquide de réglage du glissement pour régler le glissement d'une surface en caoutchouc silicone, ce liquide de réglage du glissement est utilisé comme liquide circulant à insérer dans un modèle de vaisseau sanguin pour un simulateur de cathéter en caoutchouc silicone, ce liquide de réglage du glissement comprend:
eau ;
un tensioactif cationique et/ou un tensioactif non ionique et/ou un tensioactif zwittérionique ; et
un composé ionique hydrosoluble, au nombre d'un, deux ou davantage choisis dans le groupe qui consiste en un sel de métal alcalin, un sel de métal de terre alcaline, un sel d'aluminium et un sel de fer ;
où la concentration des tensioactifs varie de 0.005 mmol/L ou davantage à 100 mmol/L ou moins ; et la concentration du composé ionique varie de 1 mmol/L ou davantage à 100 mmol/L ou moins.

2. Utilisation, selon la revendication 1, où l'agent tensioactif comprend au moins un tensioactif cationique.
